# EUROPEAN PATENT APPLICATION

(11) **EP 3 689 356 A1**
(43) Date of publication of application: **05.08.2020**
(21) Application number: 20155278.3
(22) Date of filing: 04.02.2020
(51) Int. Cl.: A61K 33/00, A23L 2/54, C02F 1/68, A61K 8/19

(54) **HYDROGEN-BASED COMPOSITIONS**

(30) Priority: 04.02.2019 AU 2019900340; 08.07.2019 AU 2019902420
(71) Applicant: Hydrogen4Health Pty Ltd, Koorlong, Victoria 3501 (AU)
(72) Inventor: Kambouris, Ambrosios, Victoria, 3501 (AU)
(74) Representative: Cabinet Chaillot

(57) **Abstract**

The present invention relates to the general field of antioxidant compositions that may confer a health benefit or an aesthetic benefit to a human. The composition may take the form of a medicament, a beverage, an infusion, or a topical. The invention further relates containers comprising the antioxidant compositions, and also containers comprising gas mixtures suitable for producing the antioxidant compositions.

## Description

### FIELD OF THE INVENTION

. The present invention relates to the general field of compositions formed from a substantially aqueous solvent and having hydrogen gases dissolved therein for use with humans and other animals. The composition may take the form of a medicament, a beverage, an infusion, or a topical. More particularly, the invention relates to compositions having a mixture of hydrogen gas and another dissolved gases. The compositions may provide benefit to the recipient in the form of a health benefit or an aesthetic benefit for example.

### BACKGROUND TO THE INVENTION

. Free radical chemical species in the body are well known to cause negative health effects when present in excessive amounts. Free radicals are typically reactive oxygen species (ROS) which may be found within a cell, within a cellular organelle, or in the interstitial fluid of a mammal. Such radicals are generated is the body endogenously often in accordance with a physiochemical or pathological state in the body.

. In nature, free radicals are neutralized by antioxidant molecules which may be endogenous in the body or ingested. A fine balance between free radical and antioxidant species is necessary for good health. In some circumstances free radicals overwhelm the body's ability to neutralize them, leading to a condition known as oxidative stress. Excess free radicals are therefore free to oxidise lipids, proteins, DNA and other biological molecules of the body. Oxidation can destroy or alter a critical biological function in a molecule thereby leading to disease.

. It is known in the art that "hydrogen water" may be used as a beverage to prevent the generation of excess ROS, and therefore oxidative stress. Hydrogen water is typically prepared by the electrolysis of water such that the water is close to saturated with molecular hydrogen (to around 1.6 ppm). While useful, hydrogen water is expensive to make by electrolysis requiring large amounts of energy, and the process is generally not achievable in a domestic setting. Furthermore, electrolysis of water other than distilled water, results in chlorine gas formation due to the presence of salt. Typically, consumers purchase hydrogen water in commercial pre-packaged form. Even in industrial settings, electrolysis would be not generally feasible.

. Furthermore, hydrogen water has a limited ability to provide a desired biological effects in the body given the low levels of saturation of the gas in water. In some circumstances, a desired biological effect may only be achieved by rapidly consuming large amounts of hydrogen water with hydrogen gas levels that are much higher than the typical saturation (1.6mg/L) at standard temperature and pressure (STP). In other circumstances, the desired effect is simply not achievable.

. It is an aspect of the present invention to provide an improvement to prior art compositions that seek to reduce oxidative stress in the body, and methods of making same. It is a further aspect of the prior art to provide a useful alternative to prior art compositions and methods of making same.

. The discussion of documents, acts, materials, devices, articles and the like is included in this specification solely for the purpose of providing a context for the present invention. It is not suggested or represented that any or all of these matters formed part of the prior art base or were common general knowledge in the field relevant to the present invention as it existed before the priority date of each provisional claim of this application.

### SUMMARY OF THE INVENTION

. In a first aspect, but not necessarily the broadest aspect, the present invention provides a composition comprising a substantially aqueous solvent, the solvent having dissolved therein carbon dioxide gas and hydrogen gas. In some embodiments the hydrogen gas is above the amount achievable at saturation at standard temperature and pressure (STP) such as about 1.6mg/L. The carbon dioxide gas and hydrogen gas may be present in the composition as a result of contacting the substantially aqueous solvent with a gas mixture comprising both carbon dioxide gas and hydrogen gas.

. In one embodiment of the first aspect, the hydrogen gas is present in an amount that is sufficient to confer a health benefit on a mammal that ingests the composition.

. In one embodiment of the first aspect, the health benefit is the reduction of oxidative stress on a subcellular compartment or organelle of a cell, or a cell, or a tissue or an organ of the mammal that ingested the composition. In other embodiments, the health benefit is an upregulation of ghrelin or ghrelin-like molecule in the animal.

. In one embodiment of the first aspect, the hydrogen gas is present in an amount of at least about 0.1%, 0.2%, 0.3%, 0.4%, 0.5%, 0.6%, 0.7%, 0.8%, 0.9%,. 1.0%, 1.5%, 2.0%, 2.5%, 3.0%, 3.5%, 4.0%, 4.5% or 5% by volume.

. In one embodiment of the first aspect, the carbon dioxide gas is present in an amount of at least about 0.25, 0.5, 1.0, 1.5, 2.0, 2.5, 3.0, 3.5, 4.0, 4.5, 5.0, 5.5, 6.0, 6.5, 7.0, 7.5, 8.0, 8.5, 9.0, 9.5 or 10 volumes.

. In one embodiment of the first aspect, the dissolved carbon dioxide gas leaves the substantially aqueous solvent (optionally in the form of bubbles) when the composition is exposed to room temperature and/or atmospheric pressure or STP.

. In one embodiment of the first aspect, the composition is present in a substantially hydrogen gas impermeable container, wherein a headspace comprising carbon dioxide gas and hydrogen gas at a supra-atmospheric pressure is present within the container so as to inhibit the dissolution of carbon dioxide gas and hydrogen gas.

. In one embodiment of the first aspect, the supra-atmospheric pressure is sufficient so as to allow dissolution of the carbon dioxide gas and/or hydrogen gas in an amount of greater than about 1%, 5%, 10%, 15%, 20%, 25%, 30%, 35%, 40%, 45%, 50%, 60%, 65%, 70%, 75%, 80%, 85%, 90%, 95%, or 100% compared with the amount of the carbon dioxide gas and/or hydrogen gas that is dissolved under conditions of atmospheric pressure and at the same temperature.

. In one embodiment of the first aspect, the container is fabricated from a metal.

. In one embodiment of the first aspect, the container is a can, a bottle, or a jar.

. In one embodiment of the first aspect, the can has a ring-pull opening, or the bottle has a screw cap, or the jar has a screw cap.

. In one embodiment of the first aspect, container is configured to contain a gas at a pressure of at least about 50, 100, 150, 200, 250, or 300 kPa.

. In one embodiment of the first aspect, the composition is formulated as a beverage, a medicament, an infusion, or a topical.

. In a second aspect, the present invention provides a reusable container configured to contain a pressurized gas, the container comprising a mixture of hydrogen gas and carbon dioxide gas, and a port suitable for connection to a gas input of a domestic beverage dispensing apparatus capable of carbonating a substantially aqueous solvent.

. In one embodiment of the second aspect, the reusable container is configured to be emptied and refilled with a mixture of hydrogen gas and carbon dioxide gas.

. In one embodiment of the second aspect, the apparatus capable of carbonating a substantially aqueous solvent is a domestic beverage carbonation and beverage dispensing apparatus such as a Soda Stream™ apparatus or similar.

. In one embodiment of the second aspect, the reusable container of any one of claims 14 to 16 having a weight (when filled with hydrogen gas and carbon dioxide gas) of less than about 10, 9, 8 ,7, 6, 5, 4, 3, 2 or 1 kg.

. In one embodiment of the second aspect, the apparatus capable of carbonating a substantially aqueous solvent is an industrial scale beverage carbonation apparatus.

. In one embodiment of the second aspect, the reusable container has a weight (when filled with hydrogen gas and carbon dioxide gas) of greater than about 10, 20, 30, 40, 50, 60, 70, 80, 90 or 100 kg.

. In a third aspect, the present invention provides a system for the preparation of a composition, the system comprising:
the reusable container of any embodiment of the second aspect, and
an apparatus capable of carbonating a substantially aqueous solvent,
wherein the reusable container and the apparatus capable of carbonating a substantially aqueous solvent are connectable such that gas in the reusable container is supplied to the apparatus capable of carbonating a substantially aqueous solvent.

. In a fourth aspect, the present invention provides a method for the preparation of a composition, the method comprising the steps of:
providing a substantially aqueous solvent, and
contacting the substantially aqueous solvent with carbon dioxide gas and hydrogen gas so as to dissolve the carbon dioxide gas and hydrogen gas in the substantially aqueous solvent.

. In one embodiment of the fourth aspect, the method is configured to produce the composition of any embodiment of the first aspect.

. In one embodiment of the fourth aspect, the step of contacting is performed by an apparatus capable of carbonating a substantially aqueous solvent.

. In one embodiment of the fourth aspect, the apparatus capable of carbonating a substantially aqueous solvent is a beverage dispensing apparatus such as a Soda Stream™ apparatus or similar.

. In one embodiment of the fourth aspect, the apparatus capable of carbonating a substantially aqueous solvent is beverage is an industrial scale apparatus.

. In a fifth aspect, the present invention provides a method for preventing or treating a disorder associated with oxidative stress and/or a deficiency of ghrelin or a ghrelin-like molecule, the method comprising the step of administering to a subject in need thereof an effective amount of a composition of any embodiment of the first aspect.

. In a sixth aspect, the present invention provides a method of manufacturing a medicament or a functional food for preventing or treating a disorder associated with oxidative stress and/or a deficiency of ghrelin or a ghrelin-like molecule, the method comprising the step of adding a pharmaceutically acceptable excipient or a food additive to the composition of any embodiment of the first aspect.

### DETAILED DESCRIPTION OF THE INVENTION AND PREFERRED

### EMBODIMENTS THEREOF

. After considering this description it will be apparent to one skilled in the art how the invention is implemented in various alternative embodiments and alternative applications. However, although various embodiments of the present invention will be described herein, it is understood that these embodiments are presented by way of example only, and not limitation. As such, this description of various alternative embodiments should not be construed to limit the scope or breadth of the present invention. Furthermore, statements of advantages or other aspects apply to specific exemplary embodiments, and not necessarily to all embodiments covered by the claims.

. Throughout the description and the claims of this specification the word "comprise" and variations of the word, such as "comprising" and "comprises" is not intended to exclude other additives, components, integers or steps.

. Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may.

. The present invention is predicated at least in part on the inventors' discovery that a composition comprising dissolved molecular (H₂) hydrogen gas in combination with dissolved carbon dioxide gas (CO₂) is advantageous for use by a human (or other mammal) in so far as the combination of dissolved gases is capable of exerting desirable biological effects in a recipient subject.

. It has been found firstly that the carbon dioxide and hydrogen act together (and in some circumstances may act synergistically) to increase levels of ghrelin in the recipient. Ghrelin is an endogenous hormone which regulates energy balance, amongst other physiological processes. Ghrelin signals the availability of nutrients in the digestive tract to the central nervous system, acting mainly through hypothalamic mediators. The hormone promotes both neuro-endocrine and inflammatory signals which may act to increase skeletal muscle growth, decreases protein breakdown, and increases lipolysis. Thus beverages or medicaments comprising both hydrogen and carbon dioxide may be used to improve body composition by increasing ratio of muscle to fat.

. An increases in the level of ghrelin may also be used in clinical applications to treat or prevent sarcopenia, Parkinson's disease, cachexia in chronic heart failure, COPD, cancer, endstage- renal-disease or cystic fibrosis, frailty in elderly, gastrointestinal motility disorders (e.g., gastroparesis, functional dyspepsia, postoperative ileus), after curative gastrectomy, anorexia nervosa, growth hormone deficient patients, alcohol craving, sleep-wake regulation (as noted in major depression), or sympathetic nervous activity in obesity.

. Animal research and some clinical studies have shown that ghrelin may have neuroprotective and cognitive enhancing activities, positive impacts on vascular health, anti-inflammatory activities useful in autoimmune disorders, and is markedly hepatoprotective.

. As will be appreciated, water is generally saturable by any given gas species. Thus, where hydrogen gas alone is used, only a limited upregulation of ghrelin in the recipient is possible because the water has become saturate with hydrogen. The addition of a second gas species (i.e. carbon dioxide) as a ghrelin upregulator allows for the composition to have an even greater ability to stimulate ghrelin production in the recipient. Thus, for a given volume of composition the present invention (comprising use of carbon dioxide and hydrogen) is able to induce higher levels of ghrelin than a composition having only a single dissolved gas species. Accordingly, an improved clinical effect may be noted where compositions of the present invention are administered to a recipient.

. The present invention may be embodied in a method for the treatment or prophylaxis of a condition for which increased levels of gherlin are efficacious. The condition may be any of those listed *supra,* or indeed any other condition known at the filing date of this application to be preventable or treatable by increasing the endogenous levels of gherlin or a gherlin-like molecule in the body. A condition treatable or preventable by the method may be discovered at some time in the future, with such conditions being included within the ambit of the present methods.

. In some circumstances, the present compositions may be used in respect of non-medical conditions so as to improve a subject's appearance, appetite, body composition (e.g. fat to muscle ratio), mood or other parameter.

. A further finding is that compositions of the present invention may be prepared economically and freshly in the home using a domestic carbonation apparatus, such as a by way of a soda syphon, of which many types are already known to the skilled person. Exemplary type of soda siphon are of the variety marketed by SodaStream International Limited (Israel). Thus, to achieve the beneficial biological effects provided by the combination of hydrogen and carbon dioxide a consumer may purchase a small capacity gas cylinder (such as a soda bulb having sufficient gas for a single bottle of composition, or a larger cylinder for preparing multiple bottles) and inject the combination of gases into water. The resultant water (being a composition of the present invention) may be immediately consumed, or sealed so as to preserve at least to some extent the dissolved gases for a period of time for later consumption.

. With regard to the production of a composition of the present invention using a soda siphon, a reusable container of gas may provide the source of a hydrogen and carn dioxide gas mixture. The reusable container delivers both carbon dioxide and hydrogen gas (under pressure) to a beverage dispensing apparatus, such as a soda siphon. Delivery of the gas mixture generates a biologically active beverage as described herein. The beverage may be prepared economically and freshly in the home using a domestic carbonation apparatus. In particular, the hydrogen component tends to oxidise when in solution and so maximum anti-oxidant effect is obtained where the beverage is consumed shortly after production in the home.

. To achieve the beneficial biological effects provided by the combination of hydrogen and carbon dioxide a consumer may purchase a small capacity gas cylinder (such as a soda bulb having sufficient gas mixture for a single bottle of composition, or a larger cylinder for preparing multiple bottles) and inject the gas mixture into water. When the gas cylinder is depleted, it is refilled with a mixture of hydrogen and carbon dioxide gas for reuse.

. In the absence of the present reusable container, a consumer seeking to prepare a beverage containing dissolved hydrogen would be required to use a pressurised cylinder of pure hydrogen in order to prepare a hydrogen-based beverage.

. As will be appreciated, pure hydrogen gas is extremely flammable and the need to discharge a container of pressurised hydrogen gas in a domestic situation would be an unacceptable risk. For example, when injecting hydrogen gas into a beverage there may be a slight leak at the connection between the container output port and the dispenser input port allowing gas to escape. In the home, the injection of gas would typically be performed in the kitchen, and the consumer may at the same time be cooking over a gas flame. The gas hydrogen gas may ignite in the atmosphere leading to burns or a fire. Worse, the ignition may travel back to the pressurised container causing an explosion.

. A domestic situation is very different to an industrial setting where hydrogen gas on its own may be routinely handled. In an industrial setting, strict safety protocols will typically be routinely implemented by well trained personnel when dealing with pure hydrogen gas. The personnel may wear protective clothing, and the hydrogen may be released near fire-fighting equipment and in an appropriately ventilated environment to dissipate any hydrogen which leaks into the environment. However, even under carefully controlled conditions typically provided in industrial settings, there is nevertheless advantage in reducing the prospect of ignition from a workplace risk reduction perspective at least.

. In the present invention, the container comprises also carbon dioxide gas. Accordingly, any leakage will be a leakage of hydrogen gas mixed with carbon dioxide gas. The carbon dioxide gas acts to inhibit the uncontrolled combustion of any ignited hydrogen gas. This is a significant safety feature of the present invention is no far as it is embodied in the form of a reusable cylinder comprising a mixture of hydrogen and carbon dioxide gas.

. Of course, the two gases will eventually separate after leakage into the atmosphere (hydrogen will float and carbon dioxide sink) however they will remain mixed for at least a period of time and possibly sufficient time to prevent uncontrolled combustion when near an ignition source, such as a gas flame or a sparking electric motor.

. Advantageously, the carbon dioxide (when mixed with hydrogen) performs two real and substantial functions: firstly as a combustion retardant and secondly to carbonate a beverage. In some embodiments, the amount of carbon dioxide is just sufficient so as to only reduce the possibility of hydrogen ignition to some extent, whilst in others the ratio of carbon dioxide:hydrogen is at least about 1:1, 2:1, 3:1, 4:1, 5:1, 6:1, 7:1, 8:1, 9:1, 10:1, 11:1, 12:1, 13:1, 14:1, 15:1, 16:1, 17:1, 18:1, 19:1, 20:1, 25:1, 30:1, 35:1, 40:1, 45:1, 50:1, 60:1, 70:1, 80:1, 90:1, or 99:1. At higher ratios (say, at least about 60:1, 70:1, 80:1, or 90:1) the excess of carbon dioxide over hydrogen significantly reduces the possibility of hydrogen ignition. Even where hydrogen is ignited, the presence of excessive amounts of carbon dioxide reduces the possibility of uncontrolled combustion.

. Separately to the issue of prevention of hydrogen combustion, the present invention provides a real and substantial difference in working of any prior art means by which carbon dioxide and hydrogen are separately injected into water to produce a beverage. In separately injecting the gases, the user may freely vary the ratio of carbon dioxide to hydrogen. Such variation may be undesirable. In producing a hydrogen-based beverage using separate gas containers, the user may under-dose hydrogen (thereby lessening biological effect of the beverage) but overdose on carbon dioxide (resulting in an overly bubbly beverage causing gastric bloating). In the present invention, it is possible to fix the ratio of carbon dioxide gas and hydrogen gas such that when sufficient of the gas mixture is injected into water to produce a beverage saturated in hydrogen (for maximum biological effect), the beverage is carbonated to a desired level, and not carbonated excessively or insufficiently.

. Advantageously, a container of the present invention may be provided in two, three or more forms for selection by a user according to the level of carbonation required. A first form may have a low level of carbon dioxide and a set biologically active level of hydrogen, a second form may have a medium level of carbon dioxide and the set biologically active level of hydrogen, and a third form may have a high level of carbon dioxide and the set biologically active level of hydrogen. In the absence of the present invention, the user would be forced to independently control the level of injection of the gases to give the desired ratio. Thus, the present invention provides a difference that is real and substantial.

. Dependent on the intended use of the inventive composition, various formulations may be provided.

. Where the intended use is as an ingestible composition, the composition may be formulated as a liquid medicament or a beverage. In that regard, any one of more of the following additives may be used: a sugar, a sweetener, a colorant, a flavouring agent, a flavour enhancer, an aroma, a stabilizer, a pH adjusting agent, a preservative, a thickener, a pH buffering agent, or a salt. The skilled person having the benefit of the present specification is amply enabled to formulate a beverage based on a desired commercial use. Such formulations may be for the purpose of providing a "functional food" with regard to the general aim of upregulating ghrelin levels in the body, or providing some other desirable biological response in the subject's body. In that regard, functional additives may be added such as a vitamin, a mineral, a protein, a probiotic, or a prebiotic.

. The beverage may be packaged in a vessel that is substantially impermeable to hydrogen, such that the gas remains in solution and therefore available to the subject upon ingestion. An aluminium can may be a cost effective vessel in that regard.

. For domestic applications, a screw cap aluminium or stainless steel vessel may be used to prevent escape of hydrogen.

. In some embodiments, the headspace within the vessel overlying the composition comprises hydrogen gas under pressure (and optionally also carbon dioxide gas under pressure) so as to maintain desirable amounts of the gas in solution. The vessel may be sealed in a pressurized atmosphere, or alternatively gas leaving solution and entering the headspace may provide the required pressure.

. Where the intended use is as a topical, the composition may be formulated so as to be applied to the skin by spraying, wiping, dabbing of other skin-contacting method. Low viscosity compositions will be preferred for spray applications. The topical compositions may be formulated with a humectant or other agent to inhibit evaporation from the skin surface. The carbon dioxide present in the composition slightly lowers the pH (to between about 3 and 4) due to the presence of carbonic acid. It is proposed that the acid pH of the composition dislodges dirt and dead skin cells from the *stratum corneum* layer of the skin, thereby facilitating the passage of hydrogen into the deeper skin layers such as the *stratum granulosum* and even the dermis. Once in these deeper layers, the hydrogen acts as a scavenger of ROS, thereby decreasing the oxidative stress in the tissues. An improved appearance or tone in the skin may result, in part due to the protection from oxidation of molecules such as collagen and elastin which give the skin a youthful appearance. With ongoing use, the composition may further prevent oxidation of collagen and elastin to slow ageing the skins appearance.

. In the context of a topical dermatological composition, the composition may be prepared *de novo* in the home or in a beauty salon by contacting both hydrogen gas and carbon dioxide gas from a small cylinder to a substantially aqueous solvent, such as water.

. The solvent may be pre-formulated or the composition post-formulated to contain one or more dermatologically acceptable excipients.

. As used herein, the term "dermatologically acceptable excipient" includes without limitation any adjuvant, carrier, glidant, diluent, preservative, dye/colorant, flavor enhancer, surfactant, wetting agent, dispersing agent, suspending agent, stabilizer, isotonic agent, solvent, or emulsifier, including those approved by the United States Food and Drug Administration as being acceptable for dermatological or therapeutic use on humans, or which are known, or are suitable for use in dermatological compositions.

. A dermatologically acceptable excipient may be a buffer or a salt. As required, and with the benefit of the present specification the skilled person is enabled to decide whether or not any buffer or salt is required to provide a required pH or ionic strength for the composition. Acceptable salts include those salts which retain the biological effectiveness of the dissolved hydrogen and carbon dioxide, and which are not biologically or otherwise undesirable. Salts maybe prepared from addition of an inorganic base or an organic base to the free acid. Salts derived from inorganic bases include, but are not limited to, the sodium, potassium, lithium, ammonium, calcium, magnesium, iron, zinc, copper, manganese, aluminum salts and the like. Preferred inorganic salts are the ammonium, sodium, potassium, calcium, and magnesium salts. Salts derived from organic bases include, but are not limited to, salts of primary, secondary, and tertiary amines, substituted amines including naturally occurring substituted amines, cyclic amines and basic ion exchange resins, such as ammonia, isopropylamine, trimethylamine, diethylamine, triethylamine, tripropylamine, diethanolamine, ethanolamine, 2-dimethylaminoethanol, 2-diethylaminoethanol, dicyclohexylamine, lysine, arginine, histidine, caffeine, procaine, hydrabamine, choline, betaine, benethamine, benzathine, ethylenediamine, glucosamine, methylglucamine, theobromine, triethanolamine, tromethamine, purines, piperazine, piperidine, N-ethylpiperidine, polyamine resins and the like.

. The composition is preferably formulated so as to be on the acidic side of neutral to assist the role of carbon dioxide as means for removal of dirt and dead skin cells. The pH value may be adjusted to about 4.0 to 6.0 using an acid, such as a food acid. The acid for adjusting the pH value can be any conventionally used organic or inorganic acid or mixtures thereof, and may be citric acid.

. Useful excipients for adjusting pH, buffering or otherwise altering the ionic conditions of a composition include (by name, CAS No., ELINCS No); 1 ,6-hexanediamine 124-09-4 ,204-679-6; 2-aminobutanol, 96-20-8, 202-488-2; acetic acid, 64-19-7, 200-580-7; acetyl mandelic acid, 51019-43-3 / 7322-88-5; adipic acid, 124-04-9, 204-673-3; alstonia scholaris bark extract, 91745-20-9, 294-689-7; aluminum glycinate, 13682-92-3 / 41354-48-7; aluminum lactate, 18917-91 -4, 242-670-9; aluminum triformate, 7360-53-4,230-898-1 ; aminoethyl propanediol, 1 15-70-8, 204-101 -2; aminomethyl propanediol, 1 15-69-5, 204-100-7 ; aminomethyl propanol, 124-68-5, 204-709-8; aminopropanediol, 616-30-8, 210-475-8; ammonia, 7664-41 -7; 231 -635-3; ammonium acetate 631 -61 -8, 21 1 -162-9; ammonium bicarbonate, 1066-33-7, 213-91 1 -5; ammonium carbamate, 1 1 1 1 -78-0, 214-185-2; ammonium carbonate, 10361 -29-2, 233-786-0; ammonium chloride, 12125-02-9 , 235-186-4; ammonium glycolate 35249-89-9; ammonium hydroxide, 1336-21 -6, 215-647-6; ammonium lactate, 515-98-0, 208-214-8; ammonium molybdate 12054-85-2; ammonium nitrate 6484-52-2, 229-347-8; ammonium phosphate, 7722-76-1 , 231 -764-5; ammonium thiocyanate, 1762-95-4, 217-175-6; ammonium vanadate, 7803-55-6, 232-261 -3; ascorbic acid, 50-81 -7 / 62624-30-0, 200-066-2 / 263-644-3; azelaic acid, 123-99-9; 204-669; babassu acid; bakuhan; benzilic acid, 76-93-7, 200-993-2; bis-hydroxyethyl tromethamine, 6976-37-0, 230-237-7; bismuth citrate, 813-93-4, 212-390-1 ; boric acid, 10043-35-3 / 1 1 1 13-50-1 , 233-139-2 / 234-343-4; butyl diethanolamine, 102-79-4, 203-055-0; calcium carbonate, 471 - 34-1 , 207-439-9; calcium citrate 813-94-5, 212-391 -7; calcium dihydrogen phosphate, 7758-23-8, 231 -837-1 ; calcium glycinate, 35947-07-0, 252-809-5; calcium hydroxide, 1305-62-0; 215-137-3; calcium lactate, 814-80-2, 212-406-7; calcium oxide, 1305-78-8 , 215-138-9; calcium phosphate, 7758-23-8 / 10103-46-5, 231 -837-1 / 233-283-6; citric acid 77-92-9 / 5949-29-1 , 201 -069-1 ; clay minerals; copper glycinate, 32817-15-5, 251 -238-9; diammonium citrate, 3012-65-5, 221 -146-3; diammonium phosphate, 7783-28-0, 231 -987-8; dibutyl ethanolamine, 102-81 -8, 203-057-1 , diethyl ethanolamine, 100-37-8, 202-845-2; dimethyl isopropanolamine, 108-16-7, 203-556-4; dimethyl mea, 108-01 -0, 203-542-8; dioleoyl edetolmonium methosulfate, 1 1 1030-96-7; dioleyl phosphate, 14450-07-8,238-431 -3; dipotassium phosphate, 7758-1 1 -4, 231 -834-5; disodium fumarate, 17013-01 -3, 241 -087-7; disodium phosphate, 7558-79-4 / 7782-85-6, 231 - 448-7; disodium pyrophosphate, 7758-16-9, 231 -835-0; disodium tartrate ,868-18-8, 212-773-3; ethanolamine, 141 -43-5, 205-483-3; ethanolamine HCL, 2002-24-6, 217-900-6; ethyl ethanolamine, 1 10-73-6, 203-797-5; fumaric acid, 1 10-17-8, 203-743-0; galacturonic acid, 685-73-4, 21 1 -682-6; glucoheptonic acid, 23351 -51 -1 , 245-601 -0; gluconic acid, 526-95-4, 208-401 -4; glucuronic acid, 576-37-4; 209-401 -7; glutaric acid, 1 10-94-1 , 203-817-2; glycine, 56-40-6, 200-272-2; glycolic acid, 79-14-1 201 -180-5; glyoxylic acid, 298-12-4, 206-058-5; guanidine carbonate, 593-85-1 , 209-813-7; guanidine HCI, 50-01 -1 , 200-002-3; hydrobromic acid, 10035-10-6, 233-1 13-0; hydrochloric acid, 7647-01 -0, 231 -595-7; hydroxyectoin, 165542-15-4, 442-870-8; hydroxyethylpiperazine ethane sulfonic acid, 7365-45-9, 230-907-9; imidazole, 288-32-4, 206-019-2; isobutyric acid, 79-31 -2, 201 -195-7; isopropanolamine, 78-96-6, 201 -162-7; isopropylamine 75-31 -0200-860-9; lactic acid, 50-21 -5, 200-018-0; lactobionic acid, 96-82-2, 202-538-3; lauryl p-cresol ketoxime, 50652-76-1 ; lithium carbonate, 554-13-2, 209-062-5; lithium hydroxide, 1310-65-2, 215-183-4; magnesium acetate, 142-72-3, 205-554-9; magnesium carbonate hydroxide, 12125-28-9, 235-192-7; magnesium glycinate, 14783-68-7, 238-852-2; magnesium hydroxide, 1309-42-8, 215-170-3; magnesium lactate, 18917-93-6, 242-671 -4; magnesium oxide, 1309-48-4, 215-171 -9; maleic acid, 1 10-16-7, 203-742-5; malic acid, 97-67-6, 202-601 -5; malonic acid, 141 -82-2, 205-503-0; maltobionic acid 534-42-9; mea-borate, 10377-81 -8, 233-829-3; metaphosphoric acid, 37267-86-0, 253-433-4; methoxy peg-100/polyepsilon caprolactone ethylhexanoate; methoxypeg-100/polyepsilon caprolactone palmitate; methoxy peg-1 14/polyepsilon caprolactone; methylethanolamine, 109-83-1 , 203-710-0, monosodium citrate, 18996-35-5, 242-734-6; mudstone powder; paecilomyces japonica/g rape/cucumber juice extract ferment filtrate; pentapotassium triphosphate, 13845-36-8, 237-574-9; pentasodium triphosphate, 7758-29-4, 231 -838-7; phenolsulfonphthalein, 143-74-8, 205-609-7; phenyl mercuric borate, 102-98-7, 203-068-1 ; phosphonobutanetricarboxylic acid, 37971 -36-1 , 253-733-5; phosphoric acid, 7664-38-2, 231 -633-2; phosphorus pentoxide, 1314-56-3, 215-236-1 ; potassium bicarbonate, 298-14-6, 206-059-0; potassium biphthalate, 877-24-7, 212-889-4; potassium bitartrate, 868-14-4, 212-769-1 ; potassium borate, 1332-77-0, 215-575-5; potassium carbonate, 584-08-7, 209-529-3; potassium citrate, 866-84-2, 212-755-5; potassium hydroxide, 1310-58-3, 215-181 -3; potassium lactate, 996-31 -6 / 85895-78-9, 213-631 -3 / 288-752-8; potassium magnesium aspartate, 67528-13-6; potassium oxide, 12136-45-7, 235-227-6; potassium phosphate, 7778-77-0 / 16068-46-5, 231 -913-4 / 240-213-8; potassium sodium tartrate, 304-59-6, 206-156-8; potassium tartrate, 921 -53-9, 213-067-8; propane tricarboxylic acid, 99-14-9 / 51750-56-2, 202-733-3 ; quinic acid, 77-95-2 / 562-73-2 / 36413-60-2, 201 -072-8 / 209-233-4; ribonic acid, 17812-24-7; sebacic acid, 1 1 1 -20-6, 203-845-5; sesquiethoxytriethanolamine; sh-decapeptide-7; sodium acetate, 127-09-3, 204-823-8; sodium aluminate, 1302-42-7, 215-100-1 ; sodium aluminum lactate, 68953-69-5, 273-223-6; sodium arachidate; sodium aspartate, 17090-93-6 / 3792-50-5, 241 -155-6 / 223-264-0; sodium bicarbonate, 144-55-8, 205-633-8; sodium bisulfate, 7681 -38-1 , 231 -665-7; sodium borate, 1330-43-4 / 1303-96-4 215-540-4; sodium butoxyethoxy acetate, 67990-17-4, 268-040-3 ; sodium calcium boron phosphate; sodium calcium copper phosphate; sodium calcium zinc phosphate; sodium carbonate, 497-19-8, 207-838-8; sodium citrate, 68-04-2 / 6132-04-3, 200-675-3; sodium esylate, 5324-47-0, 226-194-9; sodium formate, 141 -53-7, 205-488-0; sodium fumarate 5873-57-4 / 7704-73-6, 227-535-4 / 231 -725-2; sodium glycolate, 2836-32-0, 220-624-9; sodium humate, 68131 -04-4; sodium hydroxide, 1310-73-2, 215-185-5; sodium lactate, 72-17-3 / 867-56-1 , 200-772-0 / 212-762-3; sodium metaphosphate, 10361 -03-2 / 50813-16-6, 233-782-9 / 256-779-4; sodium metasilicate, 6834-92-0, 229-912-9; sodium oxide, 1313-59-3, 215-208-9; sodium phosphate, 7558-80-7 / 7632-05-5, 231 -449-2 / 231 -558-5; sodium sesquicarbonate, 533-96-0, 208-580-9; sodium silicate, 1344-09-8, 215-687-4; sodium succinate, 2922-54-5, 220-871 -2; sodium trimetaphosphate, 7785-84-4, 232-088-3; strontium hydroxide 18480-07-4 / 131 1 -10-0, 242-367-1 ; succinic acid, 1 10-15-6; 203-740-4 sulfuric acid, 7664-93-9, 231 -639-5; tartaric acid, 133-37-9 / 147-71 -7 / 87-69-4 ,205-105-7 / 205-695-6 / 201 -766-0; taurine, 107-35-7, 203-483-8; tea-diricinoleate/ipdi copolymer, 351425-02-0; tea-hydroiodide 7601 -53-8, 231 -508-2; tea-sulfate, 7376-31 -0, 230-934-6; tetrahydroxyethyl ethylenediamine, 140-07-8, 205-396-0; tetrapotassium pyrophosphate, 7320-34-5, 230-785-7; tetrasodium pyrophosphate, 7722-88-5, 231 -767-1 ; triethanolamine, 102-71 -6, 203-049-8; triisopropanolamine, 122-20-3, 204-528-4; trisodium phosphate; 7601 -54-9, 231 -509-8; trisodium sulfosuccinate, 13419-59-5, 236-524-3; triticum vulgare protein, 100684-25-1 , 309-696-3; triticum vulgare seed extract, 84012-44-2, 281 -689-7; tromethamine, 77-86-1 , 201 -064-4; urea, 57-13-6, 200-315-5; uric acid, 69-93-2, 200-720-7; zinc carbonate hydroxide, 150607-22-0; zinc glycinate, 14281 -83-5, 238-173-1 ; zinc hexametaphosphate, 13566-15-9, 236-967-2; and zinc magnesium aspartate.

. Where a surfactant is included as an excipient, the surfactant can be any conventionally used anionic, cationic, nonionic, zwitterionic or amphetoric surfactant or mixtures thereof, and may be a sodium C14-16 olefin sulfonate.

. In some embodiments, the composition is formulated as an ingestible medicament and in such form may comprise one or pharmaceutically acceptable excipients or additional biologically active agents. The composition of the present invention may be formulated for oral administration by compounding to form an emulsion, suspension, elixir, syrup, or any other liquid form suitable capable of retaining hydrogen gas and carbon dioxide gas in solution.

. It will be appreciated that compositions suitable for other routes of administration such as transdermal, buccal, intrathecal, rectal, nasal, and the like are not excluded from the present invention however for reasons of convenience at least orally ingestible compositions are generally preferred. The medicament may be in the form of a liquid, a syrup, a suspension, or a slurry.

. The present compositions are typically implemented by the user by spreading, gently rubbing or massaging the composition onto the skin of an animal. In the context of the present invention the term "animal" is intended to include without limitation any mammal such as a human, primate, domestic animal, beast of burden, zoo animal, agriculturally or economically significant animal. As will be appreciated, given the aesthetic and functional advantages of the present compositions as disclosed herein it is the primary intention that the compositions are formulated so as to be useful in application to humans, and in particular the skin of the face or upper torso.

. The skin may be wetted prior to application of the composition. Typically, the skin is left untouched so as to allow for the penetration of active ingredients into the skin.

. The composition may be used in a dermatologically effective amount, which refers to that amount which, when administered dermatologically (i.e., topically) to an animal, is sufficient to effect the desired effect. The amount of composition which constitutes a dermatologically effective amount may vary depending on, the condition of the skin and the need for improvement, and the age of the animal to be treated, but can be determined routinely by one of ordinary skill in the art having regard to his own knowledge and to this disclosure.

. The present compositions may be formulated as a spray, a cream (with an aqueous or non-aqueous base, or a mixed base - oil in water or water in oil), a foam, a foaming solution, a lotion, a balm, a soap, a serum, or a cleanser.

. The present invention is not limited to compositions produced in the home as described. For example, the compositions may be prepared industrially and packaged into a bottle, can, jar or similar for dispatch to a retail outlet. The hydrogen may be added to the water by electrolysis or any other means.

. Furthermore, the carbon dioxide may be added by means other than gas contact methods, such as natural carbonation as a result of fermentation. For example, the beverage may be formulated as a "kombucha", being a probiotic-fermented tea beverage, combining tea, water, sugar and kombucha bacteria cultures and yeast. The bacteria and yeast ferment the sugar, yielding an effervescent drink that is low in sugar. The beverages often incorporate herb and fruit flavors to provide a variety of tastes.

. Alternatively or additionally, the use of salts such as sodium bicarbonate with an acid such as citric acid may be used to create a carbon dioxide effervescence.

. Compositions having various levels of hydrogen and carbon dioxide gas are recited in the summary of invention section herein are independently selectable. For some applications, the composition may comprise a relatively high amount of carbon dioxide and a relatively low amount of hydrogen, or a relatively low amount of carbon dioxide and a relatively high amount of hydrogen. In this context, the relativity of the amount is with regard to the saturable amount of the gas concerned.

. As used herein, the term "treatment", "treat" or "treating" as used herein means alleviating, inhibiting, slowing or arresting the development, reversing and/or relieving a condition related to alcohol consumption (directly caused by alcohol consumption, or secondary to alcohol consumption) including any disease, disorder, state, syndrome, symptom or aesthetic condition to which such term is associated. It is not intended that these terms are used to indicate a complete cure of the condition.

. The term "prophylaxis", "prevent" or "prevention" as used herein means preventing of the development of, or alleviating to some extent, a condition related to alcohol consumption (directly caused by alcohol consumption, or secondary to alcohol consumption) including any disease, disorder, state, syndrome, symptom or aesthetic condition to which such term is associated. It is not intended that these terms are used to indicate a complete prevention of the condition.

. The term effective amount" as used herein means an amount of the active compound(s) as described herein sufficient to effect beneficial or desired results for treating or preventing a condition, including any disease, disorder, state, syndrome, symptom of aesthetic condition to which such term is associated. An effective amount may be administered by way of one or more doses.

. The term "pharmaceutically acceptable" as used herein means the carrier, diluent, and /or excipients used in the composition must be compatible with the other ingredients of the formulation, and not deleterious to the recipient thereof.

. The term "pharmaceutically acceptable excipient" as used herein means a nontoxic, inert solid, semi-solid, diluent, encapsulating material or formulation auxiliary of any type. Some examples of materials which can serve as pharmaceutically acceptable excipient are sugars such as lactose, glucose, and sucrose; sweetening, flavoring, colouring and perfuming agents; preservatives according to the judgment of the formulator.

. The present invention will now be more fully described by reference to the following non-limiting Examples.

### EXAMPLE 1: Formulation of a functional beverage composition, and use thereof in a subject to improve muscle to fat ratio.

. A composition is prepared using Reverse Osmosis permeate water as a solvent, to produce a beverage with both molecular hydrogen gas and carbon dioxide, in different quantities of each present in the water beverage.

. The water, to which are added 1.472 g/L mineral mixture (Calcium Sulfate Dihydrate, 327mg/L, Calcium Carbonate 364.2mg/L, Magnesium Sulfate 663.6mg/L, Potassium Bicarbonate 3.48 mg/L, Potassium Chloride 3.6 mg/L and Sodium Chloride 109 mg/L) and placed in either a pressure vessel that can be pressurized with a standard blended gas mixture of both H₂ and CO₂ or using a domestic carbonator such as a Soda Stream™.

. One method of carbonating is forced carbonation, a process that is often used in beer making and involves knowing liquid temperature and maintaining constant gas pressure till equilibrium of gas occurs between headspace and the liquid in a sealed pressure resistant vessel. The tables for forced carbonation are publicly available.

. Forced carbonation of the mineralized water in this example, is carried out when the mineralized water is kept at 4 degrees Celsius throughout carbonation. The temperature of a liquid when lower than room temperature makes carbonation easier to achieve since the liquid can store more gas. The gas pressure used for carbonation (psi) is above STP and higher, to achieve levels of carbonation of 1.47 volume, 1.9 volumes, 3.1 volumes etc. using a gas mixture with the following blend of gasses: 2%v/v H₂ and 98% v/v CO₂.

. Table 1 shows the composition of hydrogen and carbon dioxide gas in the mineralized water, at different levels of carbonation at equilibrium.

**Table 1: Effects of carbonation on mineralized water at 10 C, at different headspace pressures till equilibrium is reached using a hydrogen: carbon dioxide gas mixture of 2% H2 & 98% CO2**

| Carbonation Temperature (° C) | Carbonation Pressure (psi) | Total gas volume in water | H₂ Gas volume In water | CO₂ Gas volume In water |
|---|---|---|---|---|
| 10 | 5 | 1.5 | 0.03 | 1.47 |
| 10 | 10 | 1.9 | 0.038 | 1.862 |
| 10 | 25 | 3.1 | 0.062 | 3.038 |

**Table 2: Effects of carbonation on mineralized water at 10 C, at different headspace pressure, till equilibrium is reached using a hydrogen: carbon dioxide gas mixture of 4% H2 & 96% CO2**

| Carbonation Temperature (° C) | Carbonation Pressure (psi) | Total gas volume in water | H₂ Gas volume In water | CO2 Gas volume In water |
|---|---|---|---|---|
| 10 | 5 | 1.5 | 0.06 | 1.44 |
| 10 | 10 | 1.9 | 0.076 | 1.824 |
| 10 | 25 | 3.1 | 0.124 | 2.976 |

. It is clear from this example that both CO2 levels and H2 gas levels, although dosed as a mixture, can be changed to suit the liquid requirements.

. In another example, a Soda Stream™ home carbonator was used to carbonate tap water that was chilled in a domestic refrigerator for several hours. In this example, the recommended gas pressure cylinder which was filled with carbon dioxide, was emptied and refilled with a gas mixture of 4% hydrogen gas and 96% carbon dioxide gas and used to carbonate the chilled tap water. In this example, carbonation at 4g/L carbon dioxide was obtained with an average molecular hydrogen gas amount of 150ppm.

. In other examples using the Soda Stream™, natural juice, colas, lemonade could all be carbonated, obtaining therapeutic levels of both hydrogen and carbon dioxide in the liquids. Using a gas mixture of 0.25% H2 v/v in 99.75% CO2 and carbonating to obtain 0.5g/L CO2 in the liquid, the H2 was nearly that of saturation i.e. 1.3ppm. In this instance, the liquid treated did not appear effervescent, nor did it taste effervescent. This was then the lowest combination of hydrogen gas in carbon dioxide gas used. Such a low level of carbonation is suitable for "still" beverages such as wine, where carbonation or effervescence is not required but hydrogen is desirable. The addition of hydrogen and carbon dioxide at this low level of addition prolonged the shelf life of wine against oxidation when compared to the same wine without hydrogen addition.

. A composition produced according to any method of this example is consumed in moderate amounts (such as 300 mL) daily to achieve the desired alteration to muscle:fat ratio in the subject.

### EXAMPLE 2: Formulation of a dermatological composition, and use thereof in a subject to improve the appearance of skin

. In an example of producing a dermatological composition to improve the skin, a SodaStream home carbonator was used to carbonate tap water. In this example, tap water was chilled in a domestic refrigerator for several hours before being carbonated using a gas cylinder filled with a blend of hydrogen gas (4%) and carbon dioxide gas (96%) until a carbonation level of 5 g/L CO2 were reached. In this example, the water contained just below 5 g/L carbon dioxide and an average molecular hydrogen gas amount of 200 ppm.

. This gasified solution was then placed in a bowl and the subjects face was repeatedly immerged into the effervescent water bath whilst bubbles escaped the liquid and interacted with the skin. After about 15 minutes, the candidate felt her face was clean and refreshed and the oily pre-treated skin has vanished to a silkier façade.

. This similar example was repeated using a 1L aerosol can filled with hydrogen gas (3%) and CO2 gas (97%). In this instance, the disposable aerosol can was attached to a diffuser apparatus that was placed in a bowl to be used to treat the face of the subject. In this instance, blended gas was released into the bowl of tap water and the bubbles were continuous throughout the 15-minute duration face bath. In this instant, the effects on the skin according to the users was more pronounced.

. This same concept of diffusing the blended gas was also tested in a bath which allowed the subject to emerge themselves in without sinking their head and furthermore, it was used to soak the subject's feet. In both these instances, satisfaction was gained.

. Subjects that were regularly using sparkling mineral water for face bathing, noted significant satisfaction when hydrogen was included into the skin baths.

. In some instances, other ingredients were added to their baths which did not deter from the fundamental hydrogen combined with carbon dioxide benefit. It appears that the carbon dioxide evolving from the liquid solution in such skin baths, physically scrubs the skin to remove deposits that may block the skin pores. Furthermore, the carbonation with CO2 gas optimizes the pH of the water to that close to the skin pH, giving the skin natural replenishment and hydration.

. The addition of hydrogen in the bath quickly diffused through the skin that is being scrubbed, giving a localized, deep anti-oxidant beauty treatment as well as then being absorbed into the blood stream for more systemic beauty effects.

. In another instant, a blend of H2 gas (2%) in CO2 (98%) was applied directly to a surgical wound three times daily for 10 days. The wound healed much faster and more completely in that time of exposure, as described by the surgeon who operated.

. Those skilled in the art will appreciate that the invention described herein is susceptible to further variations and modifications other than those specifically described. It is understood that the invention comprises all such variations and modifications which fall within the spirit and scope of the present invention.

. While the invention has been disclosed in connection with the preferred embodiments shown and described in detail, various modifications and improvements thereon will become readily apparent to those skilled in the art.

. Accordingly, the spirit and scope of the present invention is not to be limited by the foregoing examples, but is to be understood in the broadest sense allowable by law.

## Claims

1. A composition comprising a substantially aqueous solvent, the solvent having dissolved therein carbon dioxide gas and hydrogen gas.

2. The composition of claim 1, wherein the hydrogen gas is present in an amount that is sufficient to confer a health benefit on a mammal that ingests the composition.

3. The composition of claim 1 or claim 2, wherein the health benefit is the reduction of oxidative stress on a subcellular compartment or organelle of a cell, or a cell, or a tissue or an organ of the mammal that ingested the composition and/or an upregulation of ghrelin or ghrelin-like molecule in the animal.

4. The composition of any one of claims 1 to 3, wherein the hydrogen gas is present in an amount of at least about 0.1%, 0.2%, 0.3%, 0.4%, 0.5%, 0.6%, 0.7%, 0.8%, 0.9%. 1.0%, 1.5%, 2.0%, 2.5%, 3.0%, 3.5%, 4.0%, 4.5% or 5% by volume.

5. The composition of any one of claims 1 to 4, wherein the carbon dioxide gas is present in an amount of at least about 1.0, 1.5, 2.0, 2.5, 3.0, 3.5, 4.0, 4.5, 5.0, 5.5, 6.0, 6.5, 7.0, 7.5, 8.0, 8.5, 9.0, 9.5 or 10 volumes.

6. The composition of any one of claims 1 to 5, wherein the dissolved carbon dioxide gas forms bubbles in the substantially aqueous solvent when the composition is exposed to room temperature and/or atmospheric pressure.

7. The composition of any one of claims 1 to 6, present in a substantially hydrogen gas impermeable container, wherein a headspace comprising carbon dioxide gas and hydrogen gas at a supra-atmospheric pressure is present within the container so as to inhibit the dissolution of carbon dioxide gas and hydrogen, and the supra-atmospheric pressure is sufficient so as to allow dissolution of the carbon dioxide gas and/or hydrogen gas in an amount of greater than about 1%, 5%, 10%, 15%, 20%, 25%, 30%, 35%, 40%, 45%, 50%, 60%, 65%, 70%, 75%, 80%, 85%, 90%, 95%, or 100% compared with the amount of the carbon dioxide gas and/or hydrogen gas that is dissolved under conditions of atmospheric pressure and at the same temperature.

8. The composition of claim 7, wherein the container is configured to contain a gas at a pressure of at least about 50, 100, 150, 200, 250, or 300 kPa.

9. A reusable container configured to contain a pressurized gas, the container comprising
a mixture of hydrogen gas and carbon dioxide gas, and
a port suitable for connection to a gas input of an apparatus capable of carbonating a substantially aqueous solvent.

10. The reusable container of claim 9, wherein the apparatus capable of carbonating a substantially aqueous solvent is a domestic beverage carbonation and beverage dispensing apparatus such as a Soda Stream™ apparatus or similar.

11. The reusable container of claim 9 or claim 10 having a weight (when filled with hydrogen gas and carbon dioxide gas) of less than about 10, 9, 8 ,7, 6, 5, 4, 3, 2 or 1 kg.

12. The reusable container of any one of claims 9 to 12, wherein the apparatus capable of carbonating a substantially aqueous solvent is an industrial scale beverage carbonation apparatus.

13. The reusable container of claim 12 having a weight (when filled with hydrogen gas and carbon dioxide gas) of greater than about 10, 20, 30, 40, 50, 60, 70, 80, 90 or 100 kg.

14. A system for the preparation of a composition, the system comprising:
the reusable container of any one of claims 9 to 13, and
an apparatus capable of carbonating a substantially aqueous solvent,
wherein the reusable container and the apparatus capable of carbonating a substantially aqueous solvent are connectable such that gas in the reusable container is supplied to the apparatus capable of carbonating a substantially aqueous solvent.

15. A method of manufacturing a medicament or a functional food for preventing or treating a disorder associated with oxidative stress and/or a deficiency of ghrelin or a ghrelin-like molecule, the method comprising the step of adding a pharmaceutically acceptable excipient or a food additive to the composition of any one of claims 1 to 6.
